# EUROPEAN PATENT APPLICATION

(11) **EP 4 442 266 A1**
(43) Date of publication of application: **09.10.2024**
(21) Application number: 22900568.1
(22) Date of filing: 30.11.2022
(51) Int. Cl.: A61K 35/745, A61P 1/04, A23C 9/12, A23L 33/135

(54) **USE OF BIFIDOBACTERIUM LACTIS BL-99 IN PREVENTION AND/OR AMELIORATION OF GASTRITIS**

(30) Priority: 30.11.2021 CN 202111453430
(71) Applicant: Inner Mongolia Yili Industrial Group Co., Ltd., Hohhot, Inner Mongolia 010110 (CN); Inner Mongolia Dairy Tech Res Institute Co Ltd, Hohhot, Inner Mongolia 010110 (CN)
(72) Inventor: ZHAO, Wen, Hohhot City, Inner Mongolia 010000 (CN); HUNG, Wei-Lian, Hohhot City, Inner Mongolia 010000 (CN); LIU, Wei-Hsien, Hohhot City, Inner Mongolia 010000 (CN); LIU, Fudong, Hohhot City, Inner Mongolia 010000 (CN); HOU, Baochao, Hohhot City, Inner Mongolia 010000 (CN); ZHANG, Haibin, Hohhot City, Inner Mongolia 010000 (CN); YIN, Xiaojing, Hohhot City, Inner Mongolia 010000 (CN); XUE, Jiangang, Hohhot City, Inner Mongolia 010000 (CN)
(74) Representative: Dehns
(86) International application number: PCT/CN2022/135672
(87) International publication number: WO 2023/098764

(57) **Abstract**

The use of *Bifidobacterium lactis* BL-99 in the prevention and/or amelioration of gastritis. The present invention provides the use of *Bifidobacterium lactis* in the preparation of a composition for preventing and/or ameliorating gastritis, wherein the *Bifidobacterium lactis* is *Bifidobacterium lactis* having the deposit number of CGMCC No. 15650. The *Bifidobacterium lactis* can prevent and/or ameliorate chronic gastritis, and/or prevent and/or ameliorate gastric mucosal atrophy associated with gastritis.

## Description

### Technical Field

The present invention relates to the new use of *Bifidobacterium lactis* BL-99 (deposit number of CGMCC No. 15650), and in particular, the present invention relates to the use of *Bifidobacterium lactis* BL-99 in the preparation of a composition for use in the prevention and/or amelioration of gastritis.

### Background Art

Chronic gastritis is a common disease of the digestive tract system, often combined with peptic ulcer, one of the precancerous diseases of gastric cancer. In recent years, the prevalence of chronic gastritis has been increasing year by year with the rise in living standards and work pressure. The clinical symptoms of the patients include epigastric discomfort, fullness, anorexia, sour regurgitation, abdominal distending pain, etc., and some of the patients may also have general psychiatric symptoms such as fatigue, emaciation, anxiety and depression. Relevant clinical studies have demonstrated that reversing the progression of chronic gastritis is also a key to preventing the occurrence of gastric cancer.

The detection of serum pepsinogen (PG) I, II and gastrin-17 may help to determine the presence or absence and degree of gastric mucosal atrophy. Serum PGI, PGII and PGI/PGII ratio in combination with the detection of anti-H.pylori antibody is helpful for risk stratification and management of chronic gastritis.

PG levels reflect the functional status of the gastric mucosa, and when gastric mucosal atrophy occurs, PGI and PGII levels decrease, more obviously PGI level decreases, and thus the PGI/PGII ratio decreases. The determination of PG helps to determine the extent of atrophy. Those having gastric atrophy have reduced PGI, reduced PGI/PGII ratio and increased serum gastrin-17 level; those having gastric antral atrophy have reduced serum gastrin-17 level and normal PGI and PGI/PGII ratio; and those having total gastric atrophy have reduced PGI, reduced PGI/PGII ratio and reduced serum gastrin-17 level. A PGI level ≤ 70 g/L and a PGI/PGII ratio ≤ 3.0 are generally used as diagnostic thresholds for atrophic gastritis, also as criteria for screening population at high risk of gastric cancer, and widely used in Europe and Japan for screening population at gastric cancer risk. In China, screening in areas with a high incidence of gastric cancer often uses the criteria of a PGI level ≤70 g/L and a PGI/PGII ≤7.0, currently as evidenced by the lack of large-sample follow-up data.

Several studies have demonstrated that the detection of serum PG is beneficial to risk stratification of the population at high risk of gastric cancer, and those who are diagnosed with atrophy by the detection of PG and those who are diagnosed with negative atrophy by the detection of PG but have a lower PGI/PGII ratio, have a higher risk of gastric cancer. Further gastroscopy should be performed. Serum PG in combination with the detection of serum anti-H.pylori antibody can divide the population into 4 groups, A, B, C and D, the incidence of gastric cancer in different groups is different, which is a valuable prediction indicator of the gastric cancer risk, and this indicator is used in Japan as a stratification method (ABCD method) for gastric cancer risk to formulate a corresponding examination strategy. Recent domestic study results show that the combined detection of PG, gastrin-17 and anti-H.pylori antibody can stratify the risk of gastric cancer and identify high-risk individuals for gastroscopy.

The PGI/PGII ratio is negatively correlated with OLGA staging, and the lower the ratio is, the higher the stage is. A PGI/PGII ratio <_ 3.0 is used as the boundary to distinguish low-risk and high-risk OLGA stages, with a sensitivity of 77%, a specificity of 85%, a positive predictive value of 45%, and a negative predictive value of up to 96%. H. pylori infection may cause increased PGI and PGII levels, especially more obviously increased PGII level, and thus the PGI/PGII ratio decreases, and after H.pylori is eradicated, the PGI and PGII levels decrease, and the PGI/PGII ratio increases.

### Summary of the Invention

An objective of the present invention is to provide the new use of *Bifidobacterium lactis.*

According to a specific embodiment of the present invention, the present invention provides the new use of a *Lactobacillus paracasei* BL-99 strain, which was deposited in the China General Microbiological Culture Collection Center on December 18, 2017 having the deposit number of CGMCC No. 15650. The *Bifidobacterium lactis* BL-99 strain is a biological material that has been disclosed in the patent document CN 110964653 A. Studies in the prior art show that the *Bifidobacterium lactis* BL-99 is a safe probiotic strain for consumption and has gastric acid resistance and intestinal juice resistance. In addition, the *Bifidobacterium lactis* BL-99 has the effects of improving the intestinal immunity, improving the intestinal barrier integrity, inhibiting the generation of intestinal inflammatory factors, etc.

By means of experiments, the inventors of the present invention have found in studies that the *Bifidobacterium lactis* having the deposit number of CGMCC No. 15650 (i.e., *Bifidobacterium lactis* BL-99) has the efficacy of preventing and/or ameliorating gastritis, which may be specifically manifested as one or more aspects of the following efficacies: ameliorating the symptoms of functional dyspepsia; increasing the levels of PGI and PGI/PGII indicators; regulating the amount of gastric acid secreted; and/or reducing the expression of inflammatory factors IL-6 and TNF-α. Thus, it can be shown that the *Bifidobacterium lactis* BL-99 of the present invention has good potential for the prevention and/or amelioration of gastric diseases such as chronic gastritis and atrophic gastritis (gastric mucosal atrophy associated with gastritis).

Therefore, the present invention provides the use of *Bifidobacterium lactis* in the preparation of a composition for use in the prevention and/or amelioration of gastritis, wherein the *Bifidobacterium lactis* is *Bifidobacterium lactis* having the deposit number of CGMCC No. 15650 (i.e., *Bifidobacterium lactis* BL-99).

According to a specific embodiment of the present invention, in the use of the *Bifidobacterium lactis* BL-99 of the present invention, the prevention and/or amelioration of gastritis comprises one or more aspects of the following efficacies:
ameliorating the symptoms of functional dyspepsia;
increasing the levels of pepsinogen I (PGI) and/or PGI/PGII indicators;
regulating the amount of gastric acid secreted; and/or
reducing the expression of inflammatory factors IL-6 and/or TNF-α.

According to a specific embodiment of the present invention, in the use of the *Bifidobacterium lactis* BL-99 of the present invention, increasing the levels of PGI and/or PGI/PGII indicators comprises increasing pepsinogen I (PGI) secretion and/or increasing the PGI/PGII ratio.

According to a specific embodiment of the present invention, in the use of the *Bifidobacterium lactis* BL-99 of the present invention, the prevention and/or amelioration of gastritis comprises:
preventing and/or ameliorating chronic gastritis, and/or
preventing and/or ameliorating gastric mucosal atrophy associated with gastritis.

The "amelioration" of the present invention comprises the alleviation of the associated symptoms.

According to a specific embodiment of the present invention, in the use of the *Bifidobacterium lactis* BL-99 of the present invention, the composition comprises a food composition, a feed composition or a pharmaceutical composition.

According to a specific embodiment of the present invention, in the use of the *Bifidobacterium lactis* BL-99 of the present invention, the *Bifidobacterium lactis* can be used in the form of a solid or liquid bacterial preparation for the preparation of the composition.

According to a specific embodiment of the present invention, in the use of the *Bifidobacterium lactis* BL-99 of the present invention, the *Bifidobacterium lactis* can be used in the form of live bacteria or inactivated bacteria for the preparation of the composition.

According to some specific embodiments of the present invention, in the use of the *Bifidobacterium lactis* BL-99 of the present invention, the composition is a pharmaceutical composition. The pharmaceutical composition further comprises a pharmaceutically acceptable auxiliary material, and the auxiliary material comprises an excipient, a diluent, a filler and/or an absorption enhancer.

According to a specific embodiment of the present invention, in the use of the *Bifidobacterium lactis* BL-99 of the present invention, the *Bifidobacterium lactis* is used in an amount of 1.0 × 10³ CFU/day-1.0 × 10¹² CFU/day, preferably 1.0 × 10⁷ CFU/day-1.0 × 10¹¹ CFU/day.

According to some specific embodiments of the present invention, in the use of the *Bifidobacterium lactis* BL-99 of the present invention, the composition is a food composition. The food may be a fermented dairy product, a cheese, a dairy-containing beverage, a solid beverage, a tableted candy, a milk tablet, an ice cream or a dairy powder.

According to a specific embodiment of the present invention, the composition of the present invention may further comprise conventional material components in the art. For example, the pharmaceutical composition may comprise an appropriate amount of auxiliary material, and the auxiliary material may be an excipient, a diluent, a filler and/or an absorption enhancer, etc. The food composition containing the *Bifidobacterium lactis* of the present invention can be produced in accordance with the food containing *Bifidobacterium lactis* in the prior art. Depending on the needs of the recipient, the composition may be in various forms, such as a powder, a lozenge, a granulate, a microcapsule and/or a liquid preparation.

In another aspect, the present invention further provides a composition for use in the prevention and/or amelioration of gastritis, which composition comprises an effective amount of *Bifidobacterium lactis* having the deposit number of CGMCC No. 15650.

According to a specific embodiment of the present invention, the composition for the prevention and/or amelioration of gastritis of the present invention comprises a food composition, a feed composition or a pharmaceutical composition.

In another aspect, the present invention further provides a method for the prevention and/or amelioration of gastritis, which method comprises administering to a subject an effective amount of *Bifidobacterium lactis* having the deposit number of CGMCC No. 15650.

According to a specific embodiment of the present invention, the prevention and/or amelioration of gastritis comprises one or more aspects of the following efficacies:
ameliorating the symptoms of functional dyspepsia;
increasing the levels of pepsinogen I (PGI) and/or PGI/PGII indicators;
regulating the amount of gastric acid secreted; and/or
reducing the expression of inflammatory factors IL-6 and/or TNF-α.

According to a specific embodiment of the present invention, the *Bifidobacterium lactis* is administered to the subject in an amount of 1.0 × 10³ CFU/day-1.0 × 10¹² CFU/day, preferably 1.0 × 10⁷ CFU/day-1.0 × 10¹¹ CFU/day.

The present invention provides the new use of *Bifidobacterium lactis* BL-99 in the preparation of a composition for use in the prevention and/or amelioration of gastritis. By means of experiments, the present invention proves that the *Bifidobacterium lactis* BL-99 can ameliorate the symptoms of functional dyspepsia; increase the levels of PGI and PGI/PGII indicators; regulate the amount of gastric acid secreted; and/or reduce the expression of inflammatory factors IL-6 and TNF-α. Thus, it can be shown that the Bifidobacterium lactis BL-99 of the present invention has good potential for the prevention and/or amelioration of gastric diseases such as chronic gastritis and atrophic gastritis (gastric mucosal atrophy associated with gastritis).

### Brief Description of the Drawings

FIG. 1 shows that *Bifidobacterium lactis* BL-99 significantly increases pepsinogen I (PGI) secretion (panel A) and increases PGI/PGII ratio (panel B).
FIG. 2 shows that *Bifidobacterium lactis* BL-99 significantly reduces G-17 secretion in population with hyperchlorhydria.
FIG. 3 shows that *Bifidobacterium lactis* BL-99 significantly increases G-17 secretion in population with hypochlorhydria.
FIG. 4 shows that *Bifidobacterium lactis* BL-99 significantly reduces the expression of inflammatory factor IL-6.
FIG. 5 shows that *Bifidobacterium lactis* BL-99 significantly reduces the expression of inflammatory factor TNF-α.

### Detailed Description of Embodiments

In order to understand the technical features, objectives and beneficial effects of the present invention more clearly, the technical solutions of the present invention are described in detail below in conjunction with specific examples. It should be understood that these examples are only used to illustrate the present invention rather than limit the scope of the present invention.

In the examples, the reagent raw materials are commercially available, and experimental methods without specifying specific conditions are conventional methods and conditions well known in the art, or are conducted according to conditions suggested by an instrument manufacturer.

### Example 1

### 1. Experimental materials and methods

### 1.1 Strain and test samples

Probiotic BL-99 solid beverage (BL-99 powder 3.34%, maltodextrin 96.66%) for low dose of 1 × 10¹⁰ CFU/day (group E) and (BL-99 powder 16.67%, maltodextrin 83.33%) for high dose of 5 × 10¹⁰ CFU/day (group F). Referring to the formula of the solid beverage, the bacterial powder therein was replaced with an equivalent dose of maltodextrin (i.e., all maltodextrin) as a placebo group (group D).

### 1.2 Subjects

Inclusion criteria: those having one or more symptoms, namely, postprandial fullness, early satiety, upper abdominal pain, upper abdominal burning sensation, postprandial nausea or vomiting, belching, upper abdominal bloating, sour regurgitation, burning sensation, retrosternal pain or discomfort, etc., with a course of disease of more than 6 months, with onset of symptoms in the past three months.

Exclusion criteria: patients having gastric *Helicobacter pylori* infection, those having acute diarrhea, those having dyspepsia caused by severe organic lesions, patients combined with severe systemic diseases such as cardiovascular, hepatic, renal and hematopoietic system diseases, and those who are unable to accept test due to physical weakness.

### 1.3 Instruments and devices

Sigma 3K30 low-temperature high-speed centrifuge, Satorious, Germany; MS2 vortex shaker, IKA, Germany; One-tenth-thousandth electronic balance (TP-214), Denver Instrument, gas chromatography (Agilent GC-8860), gas chromatograph-mass spectrometer (GC/MS, Agilent 7200-7890B Accurate-Mass Q-TOF).

### 1.4. Design of experimental study protocol

Population: 300 people were divided into 3 groups: placebo group (group D), low dose group (group E, 10 billion CFU), and high dose group (group F, 50 billion CFU). The experimental period for the population in each group was 11 weeks, including a 7-day emptying period, an 8-week bacterial powder drinking period and a 2-week follow-up period. The bacterial powder was stored in a refrigerator at 2°C-8°C. During the drinking period, the subjects took the bacterial power after mixing it with warm water at 45°C within 1 h after lunch or dinner every day, with 1 sachet per day. Fresh stool samples and blood samples were collected from the subjects at the end of the emptying period (phase V1), at the end of the fourth week (phase V2) and the end of the eighth week (phase V3) of the drinking period, and at the end of the two-week follow-up period (phase V4), respectively, and the clinical symptoms were scored (as shown in Table 1) by doctors. From the beginning to the end of the experiment, the subjects' eating habits, digestive system amelioration scores, intestinal health status scores, and defecation habit satisfaction scores were recorded every day. In the auxiliary drug intervention group, the original medication habits and personal life habits were not changed during the experiment. Throughout the experiment, the subjects were not allowed to drink any fermented food (including kimchi, pickles, fermented bean curd, fermented soybeans, cheese, active fermented dairy beverages, etc.) and other probiotic products.

**Table 1 Clinical symptom scores**

| Symptoms | Mild (1 point) | Moderate (2 points) | Severe (3 points) |
|---|---|---|---|
| Abdominal pain | Short-duration pain, without needing medication | Long-duration pain, more than 4 hours a day, still tolerable | Severe and persistent pain, with needing medication to relieve |
| Belching | Occasional onset | Often onset, causing discomfort in both flanks | Frequent onset, causing pain in both flanks |
| Early satiety | Rare/very mild | Occasional/mild | Often/severe, affecting normal life |
| Sour regurgitati on | Occasional acid regurgitation | Acid regurgitation due to improper diet | Frequent acid regurgitation |
| Abdominal burning sensation | Rare/very mild burning sensation | Occasional/mild burning sensation | Often/severe, affecting normal life |
| Nausea or vomiting | Rare/very mild vomiting | Occasional/mild vomiting | Often/severe, affecting normal life |
| Abdominal bloating | Abdominal bloating that is more severe in a short time | Abdominal bloating that is more severe and does not relieve for a long time | Abdominal bloating throughout the day |

### 1.5 Detection indicators

### 1.5.1 Analysis of serum samples

5 mL of whole blood from the subject was placed at room temperature for about 2 h and centrifuged at 3500 r/min for 15 minutes, and the serum was stored at -80°C for later test.

On the day of sampling, the blood was collected on an empty stomach, labeled, and sent to a medical testing center within the specified time, and the violent shock was avoided during transportation.

### 1.5.2 Analysis of digestive ability of stomach

The concentration of gastrin (G-17) in the serum was detected using an ELISA kit, the concentrations of pepsinogen I (PG I) and pepsinogen II (PG I I) in the serum were detected using an immunoturbidimetry kit, and finally the PG I /PG II ratio (PGR) was calculated by a calculation method. The efficacy of BL-99 on the digestive ability of stomach in the population was determined by detecting the indicators for digestive ability of stomach.

### 1.5.3 Analysis of cytokines

The cytokines IL-6 and TNF-α in the serum were detected using an ELISA kit (excell-bio).

### 2. Statistical analysis of data

Statistical analysis was performed using SPSS 20.0 and GraphPad Prism 8. To describe the sociodemographic characteristics of the subjects, descriptive statistics (percentages, means and standard deviations) were used. Intra-group comparisons were performed using independent t test, repeated measures analysis of variance (ANOVA) test or Mann-Whitney test, inter-group comparisons were performed using Kruskal-Wallis test, and enumeration data comparisons were performed using chi-square test and chi-square trend test, respectively. *P* < 0.05 was significantly different and statistically significant.

### 3. Experimental results

### 3.1 Statistics on population experiment completion

A total of 295 people with dyspepsia were recruited in the experiment, of which 37 dropped out and 258 completed the experiment, with a dropout rate of 14.3%.

### 3.2 Statistics on basic population information

Statistics on the basic information of the subjects was performed, and there was no significant difference between the groups of the population (as shown in Table 2).

**Table 2 Statistics on basic information of subjects with functional dyspepsia**

| Basic information | Functional dyspepsia (n = 258) | | |
|---|---|---|---|
| | Group D (placebo, n = 95) | Group E (low dose, n = 85) | Group F (high dose, n = 78) |
| Age (years) | 55 (45-58) | 56 (45-58) | 57 (48-59) |
| Male (%) | 21 (22.10) | 18 (21.18) | 19 (24.36) |
| Body weight (kilograms) | 64.69 ± 13.62 | 64.36 ± 11.16 | 63.83 ± 10.62 |
| Body mass index | 24.73 ± 4.76 | 25.15 ± 4.47 | 24.95 ± 5.57 |

| | | | |
|---|---|---|---|
| Note: Data are expressed as mean±standard deviation (Mean±SD) or median (interquartile range). Gender data are expressed as a percentage (%) of the number of people; and the body mass index (BMI) is body weight (kilograms) divided by the square of height (meters). | | | |

### 3.3 Effect of BL-99 on the clinical symptom scores of the total population with dyspepsia

Statistical analysis was performed on the clinical symptom scores of the subjects before and after drinking the bacterial powder. The results are as shown in Table 3. The scores of clinical symptoms such as abdominal pain, abdominal bloating, early satiety, and nausea and vomiting of the subjects in all the three groups decrease after 8 weeks of drinking BL-99 (phase V3) compared with the phase V1. After eight weeks of intervention (phase V3), compared with the placebo group, the scores of clinical symptoms of abdominal bloating, early satiety, sour regurgitation, abdominal burning sensation, belching, and nausea and vomiting in both low dose group and high dose group decrease, the dyspeptic symptoms of abdominal burning sensation and belching can be significantly ameliorated in the BL-99 high dose group (P < 0.05), and the total score of the clinical symptoms decreases significantly (P<0.05). The belching symptom is significantly ameliorated in the high dose group compared with the low dose group. It shows that drinking BL-99 powder can significantly ameliorate the symptoms of functional dyspepsia, and drinking a high dose of BL-99 for 8 weeks has the best effect.

**Table 3 Changes in clinical symptom scores of subjects with functional dyspepsia**

| Item | Phase V1 | | | Phase V3 | | | *P* value | | |
|---|---|---|---|---|---|---|---|---|---|
| | Group D placebo (n=95) | Group E low dose (n=85) | Group F high dose (n=78) | Group D placebo (n=95) | Group E low dose (n=85) | Group F high dose (n=78) | D vs E | D vs F | E vs F |
| Total score | 11.53± 5.21 | 10.34± 4.95 | 13.69± 5.03 | 3.35± 3.36 | 2.88± 3.24 | 2.41± 2.94 | 0.26 | 0.04 | 0.36 |
| Abdominal bloating | 2.05± 0.77 | 1.94± 0.85 | 2.29± 0.74 | 0.58± 0.92 | 0.55± 0.87 | 0.44± 0.81 | 0.91 | 0.36 | 0.42 |
| Abdominal pain | 1.26± 1.20 | 0.99± 1.16 | 1.59± 1.10 | 0.21± 0.62 | 0.20± 0.60 | 0.27± 0.67 | 0.99 | 0.51 | 0.53 |
| Early satiety | 1.78± 0.99 | 1.81± 0.96 | 2.08± 0.93 | 0.49± 0.85 | 0.33± 0.75 | 0.33± 0.73 | 0.14 | 0.17 | 0.92 |
| Sour regurgitati on | 1.84± 1.02 | 1.71± 1.05 | 2.12± 0.93 | 0.53± 0.86 | 0.44± 0.81 | 0.47± 0.83 | 0.49 | 0.65 | 0.83 |
| Abdominal burning sensation | 1.56± 1.19 | 1.41± 1.14 | 2.07± 1.00 | 0.43± 0.78 | 0.29± 0.69 | 0.24± 0.62 | 0.13 | 0.07 | 0.75 |
| Belching | 1.96± 0.82 | 1.86± 0.95 | 2.13± 0.89 | 0.93± 0.98 | 0.88± 0.96 | 0.54± 0.87 | 0.77 | 0.01 | 0.02 |
| Nausea or vomiting | 1.07± 1.19 | 0.73± 1.10 | 1.45± 1.19 | 0.17± 0.54 | 0.14± 0.49 | 0.13± 0.49 | 0.78 | 0.47 | 0.65 |

Pepsinogen (PG) is derived from gastric mucosal cells and can be classified into two groups based on immunohistochemistry: pepsinogen I (PGI) and pepsinogen II (PGII), mainly secreted into the gastric lumen, with only about 1% of PG circulating in the blood. PGI is mainly secreted by basal mucosal cells, and PGII is mainly secreted by chief cells, duodenal cells and pyloric glands. In the early screening process of chronic gastritis and atrophic gastritis, increased PGI indicator and reduced PGI/PGII are the most clinically reported biological indicators, including diagnosis of gastric acid status and gastric *helicobacter pylori* clearance.

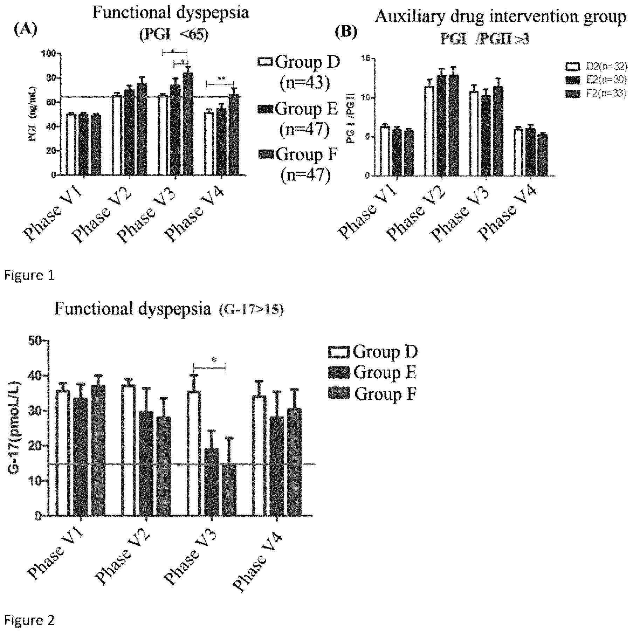

An intervention study was performed on subjects with functional dyspepsia with PGI < 50. The results are as shown in FIG. 1. The experimental results show that after 2 months of continuous intervention with *Bifidobacterium lactis* BL-99 (group E and group F), the PGI indicator significantly increases, reaching a normal level, and the change is dose-dependent. The PGI/PGII indicator also significantly increases over pre-intervention but is not significantly different compared with the placebo group (group D).

Studies show that intervention with the *Bifidobacterium lactis* BL-99 is beneficial to improving the levels of PGI and PGI/PGII indicators, and has good potential for the prevention and amelioration of gastric diseases such as chronic gastritis and atrophic gastritis.

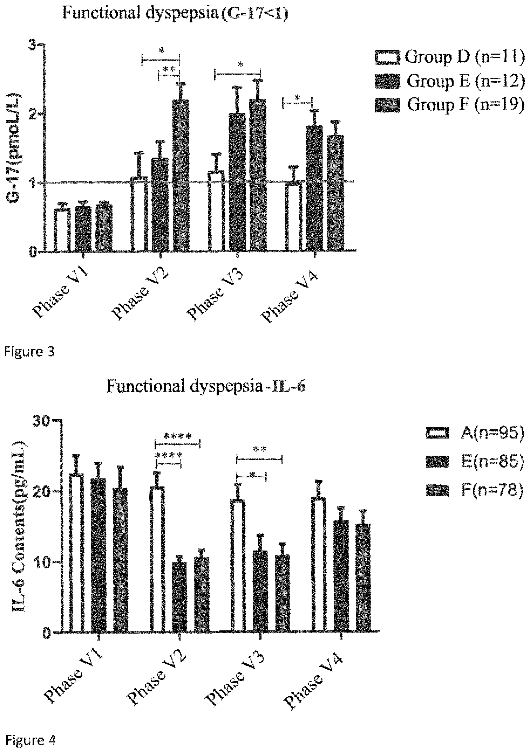

Gastrin (G-17), one of the gastrointestinal peptide hormones, can promote the release of histamine from pheochromocytoma and stimulate the secretion of a large number of gastric acid. A G-17 value higher than 15 can aggravate the occurrence of gastritis and is also one of the leading indicators of gastric cancer transformation. On the contrary, if G-17 < 1, gastric acid secretion is too low, which is manifested as symptoms such as gastric hypomotility, weakened digestive ability, early satiety, and upper abdominal fullness. Intervention with the *Bifidobacterium lactis* BL-99 (group E and group F) was performed 2 months, during which the G-17 value was detected. The results are as shown in FIG. 2 and FIG. 3. The results show that for the population with G-17 > 15, the G-17 value significantly decreases after 2 months of intervention with the Bifidobacterium lactis BL-99 (group E and group F), while for the population with too low gastric acid secretion, the G-17 value significantly returns to a normal level after one month of intervention with BL-99, which is more significant in the high dose group (group F, 50 billion CFU).

Dysregulation of gastric acid secretion is one of the main causes of chronic gastritis, and inhibition of gastric acid secretion using proton pump inhibitors such as omeprazole is also the most common clinical treatment means for chronic gastritis. However, more and more studies show that the long-term use of proton pump inhibitors can result in abnormal absorption of gastrointestinal nutrients (vitamins, minerals, etc.), damage to the gastrointestinal mucosa, increased risk of occurrence of renal and cardiovascular diseases, abnormal bone metabolism, etc. With the development of new technologies, the use of proton pump inhibitors is likely to cause disturbance of intestinal flora, and also correlates with the occurrence of Alzheimer's disease in the elderly. Based on this, the development of ingredients that can assist in reducing the amount of proton pump inhibitors used, alleviate side effects, and even replace proton pump inhibitors has broad clinical significance.

Based on this, the present invention finds that the probiotic strain *Bifidobacterium lactis* BL-99 has the effect of bidirectionally regulating the amount of gastric acid secreted.

Gastric precancerous lesion is a pathological concept, including intestinal metaplasia and dysplasia, which is an important stage in the transformation process from normal gastric tissue to gastric cancer. Gastric cancer has a high mortality rate because it is discovered after the transformation, so "inflammation-cancer transformation" is also a hot spot of clinical concern. In recent years, with the continuous research on gastric precancerous lesions, the role played by proinflammatory factors in the process has also been continuously defined.

Interleukin-6 (IL-6) play a dual proinflammatory and anti-inflammatory role in the body, and under the stimulation of inflammatory response, a large number of IL-6 can be expressed to promote the release of monocytes and neutrophils and accumulation of them in large numbers at the inflammatory site; and TNF-α is also an important mediator in the inflammatory process and mainly produced in activated monocytes and macrophages, and the expression of a large number of TNF-α also contributes to the release of a large number of other inflammatory factors. As the inflammatory response of the gastric mucosa intensifies, a large number of inflammatory factors are released and infiltrate the gastric cells, accelerating the degree of mucosal atrophy, invading the gastric mucosa, reducing the ability of intracellular glands to secrete PG, and thus affecting gastrointestinal dynamics. The inflammatory factors IL-6 and TNF-α interact with PGI, PGII, G-17, etc. to jointly affect gastric function.

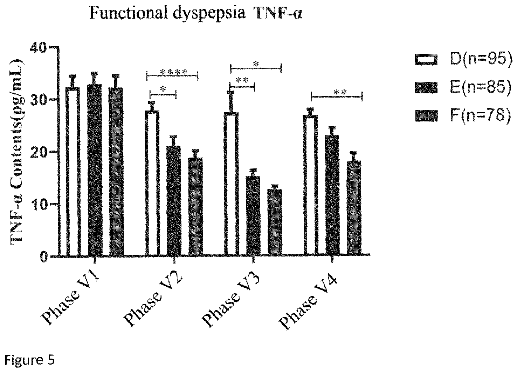

The study results show that after 1 month of intervention with the *Bifidobacterium lactis* BL-99, the expression of the inflammatory factors IL-6 (FIG. 4) and TNF-α (FIG. 5) can significantly decrease, and the degree of decrease in the inflammatory factors increases with the increase in the dose. It shows that the *Bifidobacterium lactis* BL-99 helps to reduce inflammatory factors and has the potential for the alleviation of symptoms such as gastric mucosal atrophy associated with gastritis.

## Claims

1. Use of *Bifidobacterium lactis* in the preparation of a composition for use in prevention and/or amelioration of gastritis, wherein the *Bifidobacterium lactis* is *Bifidobacterium lactis* having the deposit number of CGMCC No. 15650.

2. The use according to claim 1, wherein the prevention and/or amelioration of gastritis comprises one or more of the following efficacies:
ameliorating the symptoms of functional dyspepsia;
increasing the levels of pepsinogen I (PGI) and/or PGI/PGII indicators;
regulating the amount of gastric acid secretion; and/or
reducing the expression of inflammatory factors IL-6 and/or TNF-α.

3. The use according to claim 1, wherein the prevention and/or amelioration of gastritis comprises:
preventing and/or ameliorating chronic gastritis, and/or
preventing and/or ameliorating gastric mucosal atrophy associated with gastritis.

4. The use according to claim 1, wherein the composition comprises a food composition, a feed composition, or a pharmaceutical composition.

5. The use according to claim 1, wherein the *Bifidobacterium lactis* is used in the form of a solid or liquid bacterial preparation for the preparation of the composition.

6. The use according to claim 1, wherein the *Bifidobacterium lactis* is used in the form of live bacteria or inactivated bacteria for the preparation of the composition.

7. The use according to claim 4, wherein the pharmaceutical composition further comprises a pharmaceutically acceptable auxiliary material, and the auxiliary material comprises an excipient, a diluent, a filler, and/or an absorption enhancer.

8. The use according to any one of claims 4-7, wherein the *Bifidobacterium lactis* is taken in an amount of 1.0 × 10³ CFU/day to 1.0 × 10¹² CFU/day, preferably 1.0 × 10⁷ CFU/day to 1.0 × 10¹¹ CFU/day.

9. The use according to any one of claims 4-7, wherein the composition is a food composition.

10. The use according to claim 9, wherein the food is a fermented dairy product, cheese, a dairy beverage, a solid beverage, a tableted candy, a milk tablet, ice cream, or milk powder.

11. A composition for use in prevention and/or amelioration of gastritis, comprising an effective amount of *Bifidobacterium lactis* having the deposit number of CGMCC No. 15650.

12. The composition according to claim 11, wherein the composition comprises a food composition, a feed composition, or a pharmaceutical composition.

13. A method for prevention and/or amelioration of gastritis, comprising administering to a subject an effective amount of *Bifidobacterium lactis* having the deposit number of CGMCC No. 15650.

14. The method according to claim 13, wherein the prevention and/or amelioration of gastritis comprises one or more of the following efficacies:
ameliorating the symptoms of functional dyspepsia;
increasing the levels of pepsinogen I (PGI) and/or PGI/PGII indicators;
regulating the amount of gastric acid secretion; and/or
reducing the expression of inflammatory factors IL-6 and/or TNF-α.

15. The method according to claim 13 or 14, wherein the *Bifidobacterium lactis* is administered to the subject in an amount of 1.0 × 10³ CFU/day to 1.0 × 10¹² CFU/day, preferably 1.0 × 10⁷ CFU/day to 1.0 × 10¹¹ CFU/day.
